# EUROPEAN PATENT APPLICATION

(11) **EP 1 454 910 A1**
(43) Date of publication of application: **08.09.2004**
(21) Application number: 03290570.5
(22) Date of filing: 07.03.2003
(51) Int. Cl.: C07D 487/08, A61K 31/506, A61P 25/28, A61P 35/00

(54) **Substituted pyrimidinyl-2-(diaza-bicyclo-alkyl)-pyrimidone derivatives**

(71) Applicant: SANOFI-SYNTHELABO, 75013 Paris (FR); Mitsubishi Pharma Corporation, Osaka-shi, Osaka 541-0046 (JP)
(72) Inventor: Lochead, Alistair W., 94220 Charenton Le Pont (FR); Saady, Mourad, 75012 Paris (FR); Slowinski, Franck, 77230 Thieux (FR); Yaiche, Philippe, 93260 Les Lilas (FR)
(74) Representative: Kugel, Dominique

(57) **Abstract**

The invention relates to a 2-(diaza-bicyclo-alkyl)-pyrimidone derivative represented by formula (I) or a salt thereof: 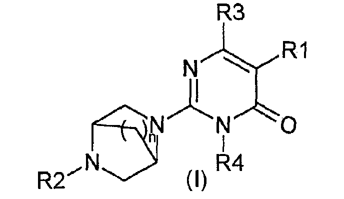 wherein:
R1 represents a hydrogen atom, a C₁₋₆ alkyl group or a halogen atom;
R2 represents a hydrogen atom, a C₁₋₆ alkyl group optionally substituted by 1 to 4 substituents selected from a halogen atom, a hydroxyl group or a C₁₋₄ alkoxy group; a C₁₋₂ perhalogenated alkyl group, a benzyl group, a phenethyl group, a benzyloxycarbonyl group, a C₁₋₄ alkoxy carbonyl group, a benzene ring, a naphthalene ring, a quinoline ring, a phthalazine ring, a 5,6,7,8-tetrahydronaphthalene ring, a pyridine ring, an indole ring, a pyrrole ring, a thiophene ring, a benzenesulfonyl group, a benzoyl group, a pyridazine ring, a furan ring or an imidazole ring; the benzyl group, the phenethyl group, the benzyloxycarbonyl group, the benzenesulfonyl group, the benzoyl group and the rings being optionally substituted by 1 to 4 substituents selected from a C₁₋₆ alkyl group, a benzene ring, a halogen atom, a C₁₋₂ perhalogenated alkyl group, a C₁₋₃ halogenated alkyl group, a hydroxyl group, a C₁₋₄ alkoxy group, a nitro, a cyano, an amino, a C₁₋₆ monoalkylamino group or a C₂₋₁₀ dialkylamino group;
R3 represents a 2, 4 or 5-pyrimidine ring optionally substituted by a C₁₋₄ alkyl group, C₁₋₄ alkoxy group or a halogen atom;
R4 represents a C₁₋₄ alkyl group optionally substituted by a hydroxyl group, a C₁₋₄ alkoxy group or a halogen atom and n represents 1 or 2.

The invention relates also to a medicament comprising the said derivative or a salt thereof as an active ingredient which is used for preventive and/or therapeutic treatment of a neurodegenerative disease caused by abnormal activity of GSK3β, such as Alzheimer disease.

## Description

### Technical Field

The present invention relates to compounds that are useful as an active ingredient of a medicament for preventive and/or therapeutic treatment of neurodegenerative diseases caused by abnormal activity of GSK3β.

### Background Art

GSK3β (glycogen synthase kinase 3β) is a proline directed serine, threonine kinase that plays an important role in the control of metabolism, differentiation and survival. It was initially identified as an enzyme able to phosphorylate and hence inhibit glycogen synthase. It was later recognized that GSK3β was identical to tau protein kinase 1 (TPK1), an enzyme that phosphorylates tau protein in epitopes that are also found to be hyperphosphorylated in Alzheimer's disease and in several taupathies. Interestingly, protein kinase B (AKT) phosphorylation of GSK3β results in a loss of its kinase activity, and it has been hypothesized that this inhibition may mediate some of the effects of neurotrophic factors. Moreover, phosphorylation by GSK3β of β-catenin, a protein involved in cell survival, results in its degradation by an ubiquitinilation dependent proteasome pathway.

Thus, it appears that inhibition of GSK3β activity may result in neurotrophic activity. Indeed there is evidence that lithium, an uncompetitive inhibitor of GSK3β, enhances neuritogenesis in some models and also increases neuronal survival, through the induction of survival factors such as Bcl-2 and the inhibition of the expression of proapoptotic factors such as P53 and Bax.

Recent studies have demonstrated that β-amyloid increases the GSK3β activity and tau protein phosphorylation. Moreover, this hyperphosphorylation as well as the neurotoxic effects of β-amyloid are blocked by lithium chloride and by a GSK3β antisense mRNA. These observations strongly suggest that GSK3β may be the link between the two major pathological processes in Alzheimer's disease: abnormal APP (Amyloid Precursor Protein) processing and tau protein hyperphosphorylation.

Although tau hyperphosphorylation results in a destabilization of the neuronal cytoskeleton, the pathological consequences of abnormal GSK3β activity are, most likely, not only due to a pathological phosphorylation of tau protein because, as mentioned above, an excessive activity of this kinase may affect survival through the modulation of the expression of apoptotic and antiapoptotic factors. Moreover, it has been shown that β-amyloid-induced increase in GSK3β activity results in the phosphorylation and, hence the inhibition of pyruvate dehydrogenase, a pivotal enzyme in energy production and acetylcholine synthesis.

Altogether these experimental observations indicate that GSK3β may find application in the treatment of the neuropathological consequences and the cognitive and attention deficits associated with Alzheimer's disease, as well as other acute and chronic neurodegenerative diseases. These include, in a nonlimiting manner, Parkinson's disease, tauopathies (e.g. frontotemporoparietal dementia, corticobasal degeneration, Pick's disease, progressive supranuclear palsy) and other dementia including vascular dementia; acute stroke and others traumatic injuries; cerebrovascular accidents (e.g. age related macular degeneration); brain and spinal cord trauma; peripheral neuropathies; retinopathies and glaucoma.

In addition GSK3β may find application in the treatment of other diseases such as: Non-insulin dependent diabetes (such as diabetes type II ) and obesity; manic depressive illness; schizophrenia; alopecia; cancers such as breast cancer, non-small cell lung carcinoma, thyroid cancer, T or B-cell leukemia and several virus-induced tumors.

Thus, it appears that inhibition of GSK3β activity may result in neurotrophic activity. Indeed there is evidence that lithium, an uncompetitive inhibitor of GSK3β, enhances neuritogenesis in some models and also increases neuronal survival, through the induction of survival factors such as Bcl-2 and the inhibition of the expression of proapoptotic factors such as P53 and Bax.

Recent studies have demonstrated that β-amyloid increases the GSK3β activity and tau protein phosphorylation. Moreover, this hyperphosphorylation as well as the neurotoxic effects of β-amyloid are blocked by lithium chloride and by a GSK3β antisense mRNA. These observations strongly suggest that GSK3β may be the link between the two major pathological processes in Alzheimer's disease: abnormal APP (Amyloid Precursor Protein) processing and tau protein hyperphosphorylation.

Although tau hyperphosphorylation results in a destabilization of the neuronal cytoskeleton, the pathological consequences of abnormal GSK3β activity are, most likely, not only due to a pathological phosphorylation of tau protein because, as mentioned above, an excessive activity of this kinase may affect survival through the modulation of the expression of apoptotic and antiapoptotic factors. Moreover, it has been shown that β-amyloid-induced increase in GSK3β activity results in the phosphorylation and, hence the inhibition of pyruvate dehydrogenase, a pivotal enzyme in energy production and acetylcholine synthesis.

### Disclosure of the Invention

An object of the present invention is to provide compounds useful as an active ingredient of a medicament for preventive and/or therapeutic treatment of a disease caused by abnormal GSK3β activity, more particularly of neurodegenerative diseases. More specifically, the object is to provide novel compounds useful as an active ingredient of a medicament that enables prevention and/or treatment of neurodegenerative diseases such as Alzheimer's disease.

Thus, the inventors of the present invention have identified compounds possessing inhibitory activity against GSK3β. As a result, they found that GRcompounds represented by the following formula (I) had the desired activity and were useful as an active ingredient of a medicament for preventive and/or therapeutic treatment of the aforementioned diseases.

The present invention thus provides 2-(diaza-bicyclo-alkyl)-pyrimidone derivatives represented by formula (I) or salts thereof, solvates thereof or hydrates thereof: 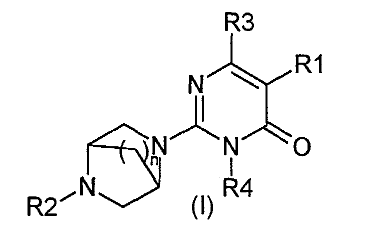 wherein:
R1 represents a hydrogen atom, a C₁₋₆ alkyl group or a halogen atom;
R2 represents a hydrogen atom, a C₁₋₆ alkyl group optionally substituted by 1 to 4 substituents selected from a halogen atom, a hydroxyl group or a C₁₋₄ alkoxy group; a C₁₋₂ perhalogenated alkyl group, a benzyl group, a phenethyl group, a benzyloxycarbonyl group, a C₁₋₄ alkoxy carbonyl group, a benzene ring, a naphthalene ring, a quinoline ring, a phthalazine ring, a 5,6,7,8-tetrahydronaphthalene ring, a pyridine ring, an indole ring, a pyrrole ring, a thiophene ring, a benzenesulfonyl group, a benzoyl group, a pyridazine ring, a furan ring or an imidazole ring; the benzyl group, the phenethyl group, the benzyloxycarbonyl group, the benzenesulfonyl group, the benzoyl group and the rings being optionally substituted by 1 to 4 substituents selected from a C₁₋₆alkyl group, a benzene ring, a halogen atom, a C₁₋₂ perhalogenated alkyl group, a C₁₋₃ halogenated alkyl group, a hydroxyl group, a C₁₋₄alkoxy group, a nitro, a cyano, an amino, a C₁₋₆ monoalkylamino group or a C₂₋₁₀ dialkylamino group;
R3 represents a 2, 4 or 5-pyrimidine ring optionally substituted by a C₁₋₄alkyl group, C₁₋₄alkoxy group or a halogen atom;
R4 represents a C₁₋₄ alkyl group optionally substituted by a hydroxyl group, a C₁₋₄ alkoxy group or a halogen atom and n represents 1 or 2.

According to another aspect of the present invention, there is provided a medicament comprising as an active ingredient a substance selected from the group consisting of the pyrimidone derivatives represented by formula (I) and the physiologically acceptable salts thereof, and the solvates thereof and the hydrates thereof. As preferred embodiments of the medicament, there are provided the aforementioned medicament which is used for preventive and/or therapeutic treatment of diseases caused by abnormal GSK3β activity, and the aforementioned medicament which is used for preventive and/or therapeutic treatment of neurodegenerative diseases and in addition other diseases such as:

Non-insulin dependent diabetes (such as diabetes type II ) and obesity; manic depressive illness; schizophrenia; alopecia; cancers such as breast cancer, non-small cell lung carcinoma, thyroid cancer, T or B-cell leukemia and several virus-induced tumors.

As further preferred embodiments of the present invention, there are provided the aforementioned medicament wherein the diseases are neurodegenerative diseases and are selected from the group consisting of Alzheimer's disease, Parkinson's disease, tauopathies (e.g. frontotemporoparietal dementia, corticobasal degeneration, Pick's disease, progressive supranuclear palsy) and other dementia including vascular dementia; acute stroke and others traumatic injuries; cerebrovascular accidents (e.g. age related macular degeneration); brain and spinal cord trauma; peripheral neuropathies; retinopathies and glaucoma, and the aforementioned medicament in the form of pharmaceutical composition containing the above substance as an active ingredient together with one or more pharmaceutical additives.

The present invention further provides an inhibitor of GSK3β activity comprising as an active ingredient a substance selected from the group consisting of the 2-(diaza-bicyclo-alkyl)-pyrimidone derivatives of formula (I) and the salts thereof, and the solvates thereof and the hydrates thereof.

According to further aspects of the present invention, there is provided a method for preventive and/or therapeutic treatment of neurodegenerative diseases caused by abnormal GSK3β activity, which comprises the step of administering to a patient a preventively and/or therapeutically effective amount of a substance selected from the group consisting of the 2-(diaza-bicyclo-alkyl)-pyrimidone derivatives of formula (I) and the physiologically acceptable salts thereof, and the solvates thereof and the hydrates thereof; and a use of a substance selected from the group consisting of the 2-(diaza-bicyclo-alkyl)-pyrimidone derivatives of formula (I) and the physiologically acceptable salts thereof, and the solvates thereof and the hydrates thereof for the manufacture of the aforementioned medicament.

As used herein, the C₁₋₆ alkyl group represents a straight or branched alkyl group having 1 to 6 carbon atoms, for example, methyl group, ethyl group, n-propyl group, isopropyl group, n-butyl group, isobutyl group, sec-butyl group, tert-butyl group, n-pentyl group, isopentyl group, neopentyl group, 1,1-dimethylpropyl group, n-hexyl group, isohexyl group, and the like;

The C₁₋₄ alkoxy group represents an alkyloxy group having 1 to 4 carbon atoms for example, methoxy group, ethoxy group, propoxy group, isopropoxy group, butoxy group, isobutoxy group, sec-butoxy group, tert-butoxy group, and the like;

The halogen atom represents a fluorine, chlorine, bromine or iodine atom;

The C₁₋₂ perhalogenated alkyl group represents an alkyl group wherein all the hydrogen have been subsituted by a halogeno, for example a CF₃ or C₂F_{5;}

The C₁₋₃ halogenated alkyl group represents an alkyl group wherein at least one hydrogen has not been substituted by an halogen atom;

The C₁₋₅ monoalkylamino group represents an amino group substituted by one C₁₋₅ alkyl group, for example, methylamino group, ethylamino group, propylamino group, isopropylamino group, butylamino group, isobutylamino group, tert-butylamino group, pentylamino group and isopentylamino group;

The C₂₋₁₀ dialkylamino group represents an amino group substituted by two C₁₋₅ alkyl groups, for example, dimethylamino group, ethylmethylamino group, diethylamino group, methylpropylamino group and diisopropylamino group;

The leaving group represents a group which could be easily cleaved and substituted, such a group may be for example a tosyl, a mesyl, a bromide and the like.

The compounds represented by the aforementioned formula (I) may form a salt. Examples of the salt include, when an acidic group exists, salts of alkali metals and alkaline earth metals such as lithium, sodium, potassium, magnesium, and calcium; salts of ammonia and amines such as methylamine, dimethylamine, trimethylamine, dicyclohexylamine, tris(hydroxymethyl)aminomethane, *N*,*N*-bis(hydroxyethyl)piperazine, 2-amino-2-methyl-1-propanol, ethanolamine, *N*-methylglucamine, and L-glucamine; or salts with basic amino acids such as lysine, - hydroxylysine, and arginine. The base-addition salts of acidic compounds are prepared by standard procedures well known in the art.

When a basic group exists, examples include salts with mineral acids such as hydrochloric acid, hydrobromic acid, sulfuric acid, nitric acid, phosphoric acid; salts with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid; or salts with acidic amino acids such as aspartic acid, and glutamic acid.

The acid-addition salts of the basic compounds are prepared by standard procedures well know in the art which include, but are not limited thereto, dissolving the free base in an aqueous alcohol solution containing the appropriate acid and isolating the salt by evaporating the solution, or by reacting the free base and an acid in an organic solvent, in which case the salt separates directly, or is precipitated with a second organic solvent, or can be obtained by concentration of the solution. The acids which can be used to prepare the acid-addition salts include preferably those which produce, when combined with the free base, pharmaceutically-acceptable salts, that is, salts whose anions are relatively innocuous to the animal organism in pharmaceutical doses of the salts, so that the beneficial properties inherent in the free base are not compromised by side effects ascribable to the anions. Although medicinally acceptable salts of the basic compounds are preferred, all acid-addition salts are within the scope of the present invention.

In addition to the 2-(diaza-bicyclo-alkyl)-pyrimidone derivatives represented by the aforementioned formula (I) and salts thereof, their solvates and hydrates also fall within the scope of the present invention. The 2-(diaza-bicycloalkyl)-pyrimidone derivatives represented by the aforementioned formula (I) may have one or more asymmetric carbon atoms. As for the stereochemistry of such asymmetric carbon atoms, they may independently be in either (R) and (S) configuration, and the derivative may exist as stereoisomers such as optical isomers, or diastereoisomers. Any stereoisomers in pure form, any mixtures of stereoisomers, racemates and the like fall within the scope of the present invention.

Examples of preferred compounds of the present invention are shown in table 1 hereinafter. However, the scope of the present invention is not limited by these compounds.

Preferred compounds of the present invention represented by formula (I) include also: Compounds wherein R3 represents a 4- or 5-pyrimidyl group and more preferably 4-pyrimidyl group, which may be substituted by a C₁₋₂ alkyl group, a C₁₋₂ alkoxy group or a halogen atom.

More preferred compounds of the present invention represented by formula (I) include also:
(1) Compounds wherein R1 represents a hydrogen atom, a C₁₋₃alkyl group or a halogen atom; more preferably a hydrogen atom; and/or
(2) Compounds wherein R2 represents a hydrogen atom; a benzyl group, a phenethyl group, a benzyloxycarbonyl group, a C₁₋₄ alkoxy carbonyl group, a benzene ring, a quinoline ring, a phthalazine ring, a pyridine ring, a benzenesulfonyl group, a benzoyl group or a pyridazine ring; the benzyl group, the phenethyl group, the benzyloxycarbonyl group, the benzenesulfonyl group, the benzoyl group and the rings being optionally substituted by 1 to 4 substituents; and/or
(3) Compounds wherein R3 represents an unsubstituted 4-pyrimidinyl group; and/or
(4) R4 represents a C₁₋₂ alkyl group preferably a methyl.

Particularly preferred compounds of the present invention represented by formula (I) include:
1: (1S)-1-Methyl-2-[5-(5-phenyl-pyridin-3-yl)-2,5-diaza-bicyclo[2.2.1]hept-2-yl]-1*H*-[4,4']bipyrimidinyl-6-one
2 : (1S)-1-Methyl-2-(5-pyridin-3-yl-2,5-diaza-bicyclo[2.2.1]hept-2-yl)-1*H*-[4,4']bipyrimidinyl-6-one
3: (1S)-1-Methyl-2-(5-quinolin-3-yl-2,5-diaza-bicyclo[2.2.1]hept-2-yl)-1*H*-[4,4']bipyrimidinyl-6-one
4 : (1R)-1-Methyl-2-(5-pyridin-3-yl-2,5-diaza-bicyclo[2.2.1]hept-2-yl)-1*H*-[4,4']bipyrimidinyl-6-one
5 : (1 S)-2-[5-(4-Fluoro-phenyl)-2,5-diaza-bicyclo[2.2.1 ]hept-2-yl]-1-methyl-1*H*-[4,4']bipyrimidinyl-6-one
6: (1R)-2-[5-(6-Chloro-quinolin-3-yl)-2,5-diaza-bicyclo[2.2.1]hept-2-yl]-1-methyl-1*H*-[4,4']bipyrimidinyl-6-one
7: (1S)-5-(1-Methyl-6-oxo-1,6-dihydro-[4,4']bipyrimidinyl-2-yl)-2,5-diazabicyclo[2.2.1]heptane-2-carboxylic acid *tert*-butyl ester
8: (1S)-2-[5-(6-Bromo-pyridin-3-yl)-2,5-diaza-bicyclo[2.2.1]hept-2-yl]-1-methyl-1*H*-[4,4']bipyrimidinyl-6-one
9: (1S)-2-[5-(6-Chloro-pyridazin-3-yl)-2,5-diaza-bicyclo[2.2.1]hept-2-yl]-1-methyl-1*H*-[4,4']bipyrimidinyl-6-one
10 : (1S)-2-[5-(5-Bromo-pyridin-2-yl)-2,5-diaza-bicyclo[2.2.1]hept-2-yl]-1-methyl-1 *H*-[4,4']bipyrimidinyl-6-one
11 : (1S)-2-[5-(4-Chloro-phthalazin-1-yl)-2,5-diaza-bicyclo[2.2.1]hept-2-yl]-1-methyl-1*H*-[4,4']bipyrimidinyl-6-one
12 : (1S)-2-[5-(4-Chloro-phenyl)-2,5-diaza-bicyclo[2.2.1]hept-2-yl]-1-methyl-1*H*-[4,4']bipyrimidinyl-6-one
13 : (1S)-2-[5-(3-Fluoro-phenyl)-2,5-diaza-bicyclo[2.2.1]hept-2-yl]-1-methyl-1*H*-[4,4']bipyrimidinyl-6-one
14 : (1S)-2-(2,5-Diaza-bicyclo[2.2.1]hept-2-yl)-1-methyl-1*H*-[4,4'] bipyrimidinyl-6-one
15 : (1S)-1-Methyl-2-(5-*p*-tolyl-2,5-diaza-bicyclo[2.2.1]hept-2-yl)-1*H*-[4,4']bipyrimidinyl-6-one
16 : (1S)-2-(5-Benzoyl-2,5-diaza-bicyclo[2.2.1]hept-2-yl)-1-methyl-1*H*-[4,4'] bipyrimidinyl-6-one
17 : (1S)-1-Methyl-2-[5-(toluene-4-sulfonyl)-2,5-diaza-bicyclo[2.2.1]hept-2-yl]-1*H*-[4,4']bipyrimidinyl-6-one
18 : (1S)-5-(1-Methyl-6-oxo-1,6-dihydro-[4,4']bipyrimidinyl-2-yl)-2,5-diazabicyclo[2.2.1 ]heptane-2-carboxylic acid benzyl ester
19 : (1S)-1-Methyl-2-(5-phenethyl-2,5-diaza-bicyclo[2.2.1]hept-2-yl)-1*H*-[4,4']bipyrimidinyl-6-one
20 : (1S)-2-(5-Benzyl-2,5-diaza-bicydo[2.2.1]hept-2-yl)-1-methyl-1 *H*-[4,4'] bipyrimidinyl-6-one
21 : (1S)-2-[5-(2(S)-Hydroxy-2-phenyl-ethyl)-2,5-diaza-bicyclo[2.2.1]hept-2-yl]-1-methyl-1*H*-[4,4']bipyrimidinyl-6-one
22 : (1S)-1-Methyl-2-(5-pyridin-2-yl-2,5-diaza-bicyclo[2.2.1]hept-2-yl)-1*H*-[4,4']bipyrimidinyl-6-one
23 : (1S)-1-Methyl-2-(5-pyridin-4-yl-2,5-diaza-bicyclo[2.2.1]hept-2-yl)-1*H*-[4,4']bipyrimidinyl-6-one
24 : (1S)-2-(5-(4-Bromo-benzoyl)-2,5-diaza-bicyclo[2.2.1]hept-2-yl)-1-methyl-1*H*-[4,4']bipyrimidinyl-6-one
25 : (1S)-2-(5-(4-Chloro-benzoyl)-2,5-diaza-bicyclo[2.2.1]hept-2-yl)-1-methyl-1*H*-[4,4']bipyrimidinyl-6-one
26 : (1S)-2-(5-(4-Fluoro-benzoyl)-2,5-diaza-bicyclo[2.2.1]hept-2-yl)-1-methyl-1*H*-[4,4']bipyrimidinyl-6-one
27 : (1S)-2-(5-(4-Methoxy-benzoyl)-2,5-diaza-bicyclo[2.2.1]hept-2-yl)-1-methyl-1*H*-[4,4']bipyrimidinyl-6-one
28: (1S)-2-(5-(4-Methyl-benzoyl)-2,5-diaza-bicyclo[2.2.1]hept-2-yl)-1-methyl-1*H*-[4,4']bipyrimidinyl-6-one
29 : (1S)-2-(5-(4-Phenyl-benzoyl)-2,5-diaza-bicyclo[2.2.1]hept-2-yl)-1-methyl-1*H*-[4,4']bipyrimidinyl-6-one
30 : (1S)-2-(5-(4-Trifluoromethyl-benzoyl)-2,5-diaza-bicyclo[2.2.1]hept-2-yl)-1-methyl-1*H*-[4,4']bipyrimidinyl-6-one
31 : (1S)-2-(5-(3-Methyl-benzoyl)-2,5-diaza-bicyclo[2.2.1]hept-2-yl)-1-methyl-1*H*-[4,4']bipyrimidinyl-6-one
32 : (1 S)-2-(5-(3-Methoxy-benzoyl)-2,5-diaza-bicyclo[2.2.1]hept-2-yl)-1-methyl-1*H*-[4,4']bipyrimidinyl-6-one
33 : (1S)-2-(5-(3-Fluoro-benzoyl)-2,5-diaza-bicyclo[2.2.1]hept-2-yl)-1-methyl-1*H*-[4,4']bipyrimidinyl-6-one
34 : (1S)-2-(5-(3-Bromo-benzoyl)-2,5-diaza-bicyclo[2.2.1]hept-2-yl)-1-methyl-1*H*-[4,4']bipyrimidinyl-6-one
35 : (1S)-2-(5-(3-Cyano-benzoyl)-2,5-diaza-bicyc)o[2.2.1]hept-2-y))-1-methyl-1*H*-[4,4']bipyrimidinyl-6-oneand
36 : (1S)-2-(5-(3-Chloro-benzoyl)-2,5-diaza-bicyclo[2.2.1]hept-2-yl)-1-methyl-1*H*-[4,4']bipyrimidinyl-6-one.

As a further object, the present invention concerns also methods for preparing the 2-(diaza-bicyclo-alkyl)-pyrimidone compounds represented by the aforementioned formula (I).

These compounds can be prepared, for example, according to methods explained below.

### Preparation method

2-(Diaza-bicyclo-alkyl)-pyrimidone compounds represented by the aforementioned formula (I), wherein R2 is as defined previously but not a hydrogen, may be prepared according to the method described in the scheme 1.

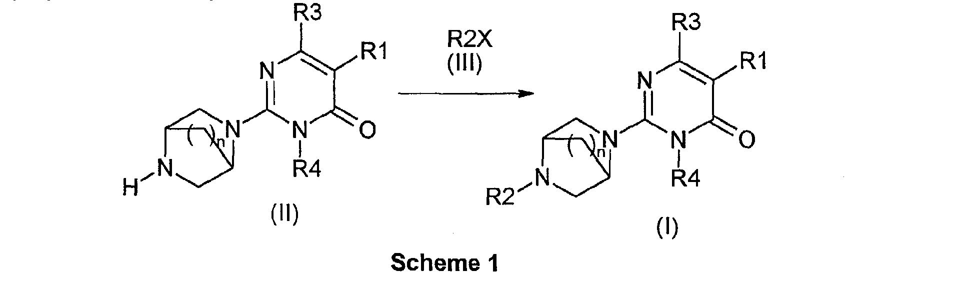

(In the above scheme the definition of R1, R3, R4 and n are the same as those already described for compound of formula (I)).

Following this method, the pyrimidinone derivative represented by the above formula (II), wherein R1, R3, R4 and n are as defined for compound of formula (I), is allowed to react, according to well known method in the art, with a compound of formula (III), wherein R2 is as defined for compound of formula (I) but not a hydrogen. For example the reaction may be carried out in a presence of a base such as alkoxide, amine or carbonate bases such as sodium *tert*-butoxide, triethylamine or cesium carbonate, in a solvent such as tetrahydrofuran or dimethylformamide and the like to give compound of formula (I).

More particularly, when R2 is an aryl group or a heteroaryl group such as defined for compound of formula (I), the reaction may be carried out according to Buchwald et al's method by a palladium-catalyzed amination (*J. Org. Chem*. **1997,** *62,* 6066-6068; *J. Am. Chem. Soc.* **1996,** *118*, 7217-7218). That is, the reaction is carried out in a presence of alkoxide, amine or carbonate bases, for example sodium *tert*-butoxide, triethylamine or cesium carbonate, and a palladium catalyst such as for example palladium(II) acetate with a ligand such as (R)-(+)-2,2'-bis(diphenylphosphino)-1,1'-binaphthyl, in a solvent such as tetrahydrofuran, dimethylformamide, tetraglyme or polyethylene glycol, at a suitable temperature ranging from 25° to 130 °C under inert atmosphere.

Alternatively, 2-(diaza-bicyclo-alkyl)-pyrimidone compounds represented by the aforementioned formula (I), wherein R2 is as defined previously for compound of formula (I), may be prepared according to scheme 2. 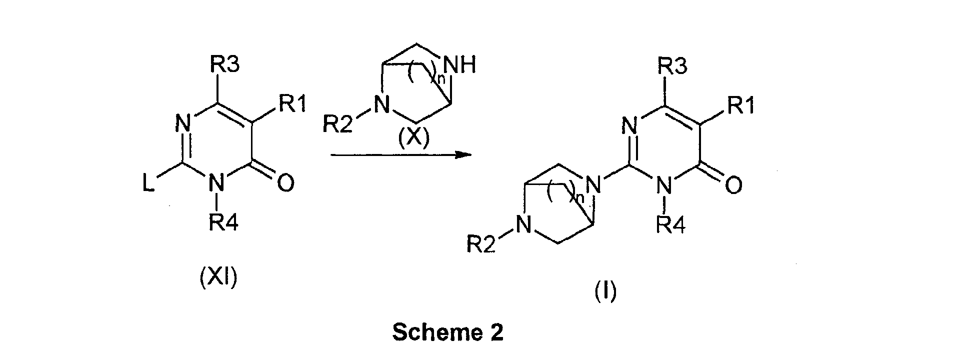 (In the above scheme the definition of R1, R2, R3, R4 and n are the same as already described for compound of formula (I)).

Following this method, compound of formula (XI), wherein R1, R3 and R4 are as defined for compound of formula (I) and L represents a leaving group such as for example a chlorine or bromine atom, is allowed to react with a compound of formula (X) wherein n and R2 are as defined for compound of formula (I). The reaction may be carried out in a presence of a base such as for example sodium hydride or triethylamine in a solvent such as dimethylformamide or tetrahydrofuran at a temperature ranging from 20° to 60°C.

The compound of formula (II) may be prepared according to the method defined in scheme 3. 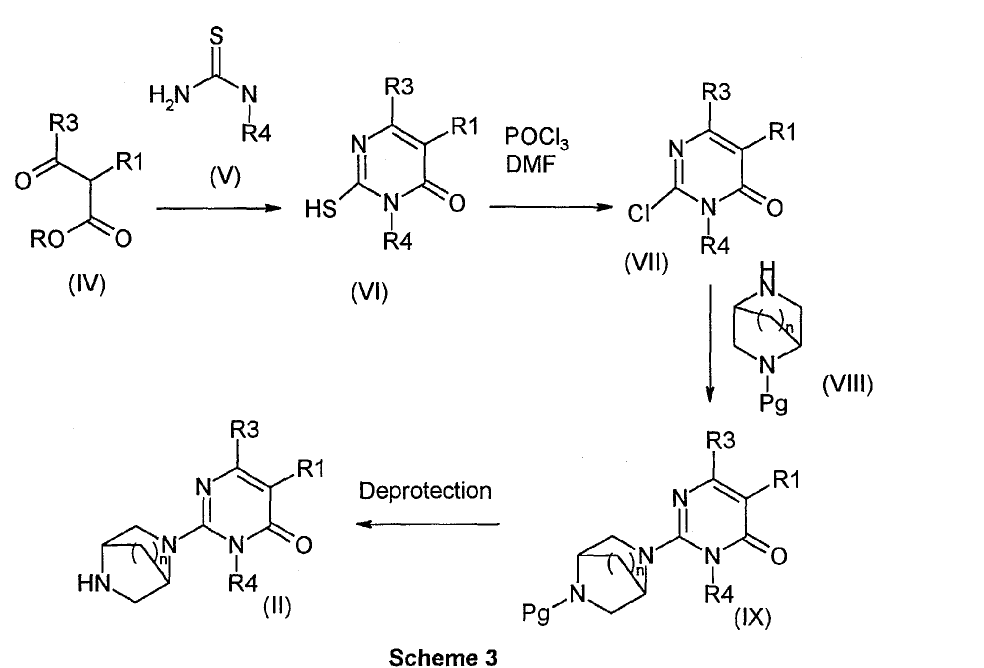 (In the above scheme the definition of R1, R3, R4 and n are the same as already described for compound of formula (I)).

According to this method, the 3-ketoester of formula (IV), wherein R1 and R3 are as defined for compound formula (I) and R is an alkyl group such as for example a methyl or ethyl group, is allowed to react with N-alkylthiourea of formula (V) wherein R4 is as defined for compound of formula (I). The reaction may be carried out in the presence of a base such as 1,8-diazabicyclo[5.4.0]undec-7-ene, in a alcoholic solvent such as ethanol, at a suitable temperature ranging from 25° to 140°C under ordinary air to give the thiopyrimidone derivative of formula (VI). The thiopyrimidone derivative of formula (VI) is allowed to react with phosphorus oxychloride in a solvent such as dimethylformamide, at a suitable temperature ranging from 0° to 55°C under argon atmosphere to give the 2-chloropyrimidone derivative of formula (VII). This latter of formula (VII) is then reacted with a compound of formula (VIII), wherein Pg is a protecting group such as for example a *tert*-butoxycarbonyl group, in the presence of a base such as triethylamine in a solvent such as tetrahydrofuran, at a suitable temperature ranging from 0° to 25°C, to give compound of formula (IX). The compound of formula (IX) is then deprotected according to well known method in the art such as for example when the protecting group is a *tert*-butoxycarbonyl group, in the presence of trifluoroacetic acid in a solvent such as dichloromethane at room temperature to give the aforementioned formula (II).

Alternatively, compounds of formula (II) wherein R1 represents a halogen atom such as a bromine atom or a chlorine atom, may be obtained by halogenation of a compound of formula (II) wherein R1 is a hydrogen atom. The reaction may be carried out in an acidic medium such as acetic acid or propionic acid, in presence of bromosuccinimide or chlorosuccinimide, or bromine.

In addition, compounds of formula (II), wherein R1 represents a fluorine atom, may be obtained by analogy to the method described in Tetrahedron Letters, Vot.30,N°45,pp 6113-6116, 1989.

Compounds of formula (III), (IV), (V), (VIII), (XI) and (X) are commercially available or may be synthesized according to well-known methods to one skilled in the art.

For example compounds of formula (IV), wherein R3 and R1 are as defined for compound of formula (I) and R is an alkyl group such as a methyl or an ethyl, can be prepared by reacting a pyrimidine-carboxylic acid optionally substituted by a C₁₋₄ alkyl group, C₁₋₄ alkoxy group or an halogen, with the corresponding malonic acid monoester. The reaction can be carried out using methods well known to one skilled in the art, such as for example in presence of a coupling agent such as 1,1'-carbonylbis-1*H*-imidazole in a solvent such as tetrahydrofuran at a temperature ranging from 20° to 70°C.

For example compounds of formula (VIII) with the absolute configuration (1R) can be prepared according to EP-400661.

In the above reactions, protection or deprotection of a functional group may sometimes be necessary. A suitable protecting group Pg can be chosen depending on the type of the functional group, and a method described in the literature may be applied. Examples of protecting groups, of protection and deprotection methods are given for example in Protective groups in Organic Synthesis Greene et al., 2nd Ed. (John Wiley & Sons, Inc., New York).

The compounds of the present invention have inhibitory activity against GSK3β. Accordingly, the compounds of the present invention are useful as an active ingredient for the preparation of a medicament, which enables preventive and/or therapeutic treatment of a disease caused by abnormal GSK3β activity and more particularly of neurodegenerative diseases such as Alzheimer's disease. In addition, the compounds of the present invention are also useful as an active ingredient for the preparation of a medicament for preventive and/or therapeutic treatment of neurodegenerative diseases such as Parkinson's disease, tauopathies (e.g. frontotemporoparietal dementia, corticobasal degeneration, Pick's disease, progressive supranuclear palsy) and other dementia including vascular dementia; acute stroke and others traumatic injuries; cerebrovascular accidents (e.g. age related macular degeneration); brain and spinal cord trauma; peripheral neuropathies; retinopathies and glaucoma; and other diseases such as non-insulin dependent diabetes (such as diabetes type II ) and obesity; manic depressive illness; schizophrenia; alopecia; cancers such as breast cancer, non-small cell lung carcinoma, thyroid cancer, T or B-cell leukemia and several virus-induced tumors.

The present invention further relates to a method for treating neurodegenerative diseases caused by abnormal activity of GSK3β and of the aforementioned diseases which comprises administering to a mammalian organism in need thereof an effective amount of a compound of the formula (I).

As the active ingredient of the medicament of the present invention, a substance may be used which is selected from the group consisting of the compound represented by the aforementioned formula (I) and pharmacologically acceptable salts thereof, and solvates thereof and hydrates thereof. The substance, per se, may be administered as the medicament of the present invention, however, it is desirable to administer the medicament in a form of a pharmaceutical composition which comprises the aforementioned substance as an active ingredient and one or more pharmaceutical additives. As the active ingredient of the medicament of the present invention, two or more of the aforementioned substances may be used in combination. The above pharmaceutical composition may be supplemented with an active ingredient of another medicament for the treatment of the above mentioned diseases. The type of pharmaceutical composition is not particularly limited, and the composition may be provided as any formulation for oral or parenteral administration. For example, the pharmaceutical composition may be formulated, for example, in the form of pharmaceutical compositions for oral administration such as granules, fine granules, powders, hard capsules, soft capsules, syrups, emulsions, suspensions, solutions and the like, or in the form of pharmaceutical compositions for parenteral administrations such as injections for intravenous, intramuscular, or subcutaneous administration, drip infusions, transdermal preparations, transmucosal preparations, nasal drops, inhalants, suppositories and the like. Injections or drip infusions may be prepared as powdery preparations such as in the form of lyophilized preparations, and may be used by dissolving just before use in an appropriate aqueous medium such as physiological saline. Sustained-release preparations such as those coated with a polymer may be directly administered intracerebrally.

Types of pharmaceutical additives used for the manufacture of the pharmaceutical composition, content ratios of the pharmaceutical additives relative to the active ingredient, and methods for preparing the pharmaceutical composition may be appropriately chosen by those skilled in the art. Inorganic or organic substances, or solid or liquid substances may be used as pharmaceutical additives. Generally, the pharmaceutical additives may be incorporated in a ratio ranging from 1 % by weight to 90% by weight based on the weight of an active ingredient.

Examples of excipients used for the preparation of solid pharmaceutical compositions include, for example, lactose, sucrose, starch, talc, cellulose, dextrin, kaolin, calcium carbonate and the like. For the preparation of liquid compositions for oral administration, a conventional inert diluent such as water or a vegetable oil may be used. The liquid composition may contain, in addition to the inert diluent, auxiliaries such as moistening agents, suspension aids, sweeteners, aromatics, colorants, and preservatives. The liquid composition may be filled in capsules made of an absorbable material such as gelatin. Examples of solvents or suspension mediums used for the preparation of compositions for parenteral administration, e.g. injections, suppositories, include water, propylene glycol, polyethylene glycol, benzyl alcohol, ethyl oleate, lecithin and the like. Examples of base materials used for suppositories include, for example, cacao butter, emulsified cacao butter, lauric lipid, witepsol.

The dose and frequency of administration of the medicament of the present invention are not particularly limited, and they may be appropriately chosen depending on conditions such as a purpose of preventive and/or therapeutic treatment, a type of a disease, the body weight or age of a patient, severity of a disease and the like. Generally, a daily dose for oral administration to an adult may be 0.01 to 1,000 mg (the weight of an active ingredient), and the dose may be administered once a day or several times a day as divided portions, or once in several days. When the medicament is used as an injection, administrations may preferably be performed continuously or intermittently in a daily dose of 0.001 to 100 mg (the weight of an active ingredient) to an adult.

### Chemical Examples

The present invention will be explained more specifically with reference to the following general examples, however, the scope of the present invention is not limited to these examples.

### Example 1 (Compound N° 7 of table 1)

### (1S)-5-(1 -Methyl-6-oxo-1,6-dihydro-[4,4']bipyrimidinyl-2-yl)-2,5-diazabicyclo[2.2.1]heptane-2-carboxylic acid tert-butyl ester. (Free base)

### 1.1. 2-Mercapto-1-methyl-1 H-[4,4']bipyrimidinyl-6-one

A mixture containing 77.0g (0.4 mol) of ethyl 3-(4-pyrimidinyl)-3-oxopropionate (prepared by analogy to the method described in patent DE 2705582), 107.0g (1.19 mol) of N-methylthiourea, 60.4g (0.4 mol) of 1,8-diazabicyclo[5.4.0]undec-7-ene in 773 ml of ethanol was heated under reflux during 2 h.
The cooled mixture was treated with 25.8ml (0.40 mol) of methanesulfonic acid diluted in 157.2 ml of water and the precipitate recovered by filtration to afford 72g of pure product as a yellow solid.
Mp : 219-221°C

### 1.2 2-Chloro-1-methyl-1H-[4,4']bipyrimidinyl-6-one

To a solution of 300 ml of dimethylformamide was added 70 ml (0.75 mol) of phosphorus oxychloride at 0°C and the resulting solution was stirred at same temperature for 15 min.
There is added 67.1 g (0.305 mol) of 2-mercapto-1-methyl-1 H-[4,4']bipyrimidinyl-6-one and the resulting solution was stirred at 55°C for 2h.
The mixture was poured into ice-water, adjusted to pH 8 with sodium hydrogen carbonate and extracted with ethyl acetate. The organic extracts were dried over sodium sulfate and evaporated to give 44.6g (66%) of the desired compound.
Mp : 150-152°C.

### 1.3 (1S)-5-(1-Methyl-6-oxo-1,6-dihydro-[4,4']bipyrimidiny)-2-yl)-2,5-diazabicyclo[2.2.1]heptane-2-carboxylic acid tert-butyl ester.

A mixture containing 1.95 ml (14 mmol) of triethylamine, 2.45g (11 mmol) of 2-chloro-1-methyl-1*H*-[4,4']bipyrimidinyl-6-one, 2.58g (13 mmol) of (1 S)-2,5-Diaza-bicyclo[2.2.1]heptane-2-carboxylic acid *tert*-butyl ester in 100 ml of anhydrous tetrahydrofuran was stirred at room temperature for 2 h.
Water was added to the cooled mixture and the resulting solution extracted with ethyl acetate. The combined extracts were washed with saturated aqueous ammonium chloride and evaporated. The crude product was purified by chromatography on silica gel eluting with a mixture of dichloromethane/methanol in the proportions 100/0 to 98/2 to afford 4.2g of pure product as a white solid.
Mp : 198-200°C

### Example 2 (Compound N°14 of table 1)

### (1S)-2-(2,5-Diaza-bicyclo[2.2.1]hept-2-yl)-1-methyl-1H-[4,4']bipyrimidinyl-6-one hydrochloride (1:1)

To a solution of 4.45g (11.58 mmol) of (1S)-5-(1-methyl-6-oxo-1,6-dihydro-[4,4']bipyrimidinyl-2-yl)-2,5-diaza-bicyclo[2.2.1]heptane-2-carboxylic acid *tert*-butyl ester in 25 ml of anhydrous dichloromethane was added 10.71 ml (139 mmol) of trifluoroacetic acid and the resulting mixture was stirred at room temperature for 2h.
The mixture was poured into ice-water, adjusted to pH 8 with potassium carbonate and extracted with chloroform. The organic extracts were dried over sodium sulfate and evaporated. The product obtained in the form of free base was transformed into the hydrochloride salt to give 4g (69%) of pure compound as a yellow solid.
Mp : 275-277°C.

### Example 3 (Compound N° 2 of table 1)

### (1S)-1-Methyl-2-(5-pyridin-3-yl-2,5-diaza-bicyclo[2.2.1]hept-2-yl)-1H-[4,4'] bipyrimidinyl-6-one hydrochloride (1:1).

A mixture containing 1.76g (6.28 mmol) of (1S)-2-(2,5-diazabicyclo[2.2.1]hept-2-yl)-1-methyl-1*H*-[4,4']bipyrimidinyl-6-one, 2.18 ml (22.67 mmol) of 3-bromopyridine, 3.44g (10.56 mmol) of cesium carbonate, 187mg (0.3 mmol) of (R)-(+)-2,2'-bis(diphenylphosphino)-1,1'-binaphthyl and 67mg (0.3 mmol) of palladium(II) acetate in 100 ml of anhydrous tetrahydrofuran under argon atmosphere was stirred under reflux for 18 h. The mixture was filtered and water was added to the mixture and the resulting solution extracted with chloroform. The combined extracts were washed with saturated aqueous ammonium chloride and evaporated. The crude product was purified by chromatography on silica gel eluting with a mixture of dichloromethane/methanol in the proportions 100/0 to 95/5. The product obtained in the form of free base was transformed into the hydrochloride salt to give 650mg (26%) of pure compound as a solid.
Mp : 180-182°C

### Example 4 (Compound N° 16 of table 1)

### 36 : (1S)-2-(5-Benzoyl-2,5-diaza-bicyclo[2.2.1]hept-2-yl)-1-methyl-1H-[4,4'] bipyrimidinyl-6-one

To a solution of 0.13g (0.46 mmol) of (1S)-2-(2,5-diaza-bicyc(o[2.2.1]hept-2-yl)-1-methyl-1*H*-[4,4']bipyrimidinyl-6-one in 3 ml of anhydrous dimethylformamide was added 24mg (0.6 mmol) of sodium hydride and the resulting mixture was stirred at 0°C for 15 min.
There is added 0.07 ml (0.6 mmol) of benzoyl chloride and the resulting solution was stirred at 0°C for 2h.
Water was added to the mixture and the resulting solution extracted with ethyl acetate. The combined extracts were washed with saturated aqueous ammonium chloride and evaporated. The crude product was purified by chromatography on silica gel eluting with a mixture of dichloromethane/methanol in the proportions 100/0 to 97/3 to give 90mg (50%) of pure compound as a solid.
Mp: 133-135°C
A list of chemical structures and physical data for compounds of the aforementioned formula (I) illustrating the present invention is given in table 1. The compounds have been prepared according to the methods of the example.

In the table 1, (S) or (R) indicate the stereochemistry of the carbon atom, R3 is a 4-pyrimidinyl group and R4 is a methyl group.

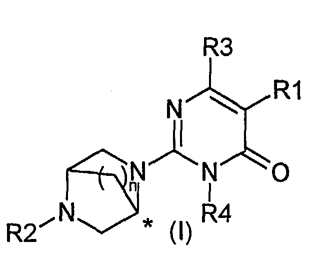

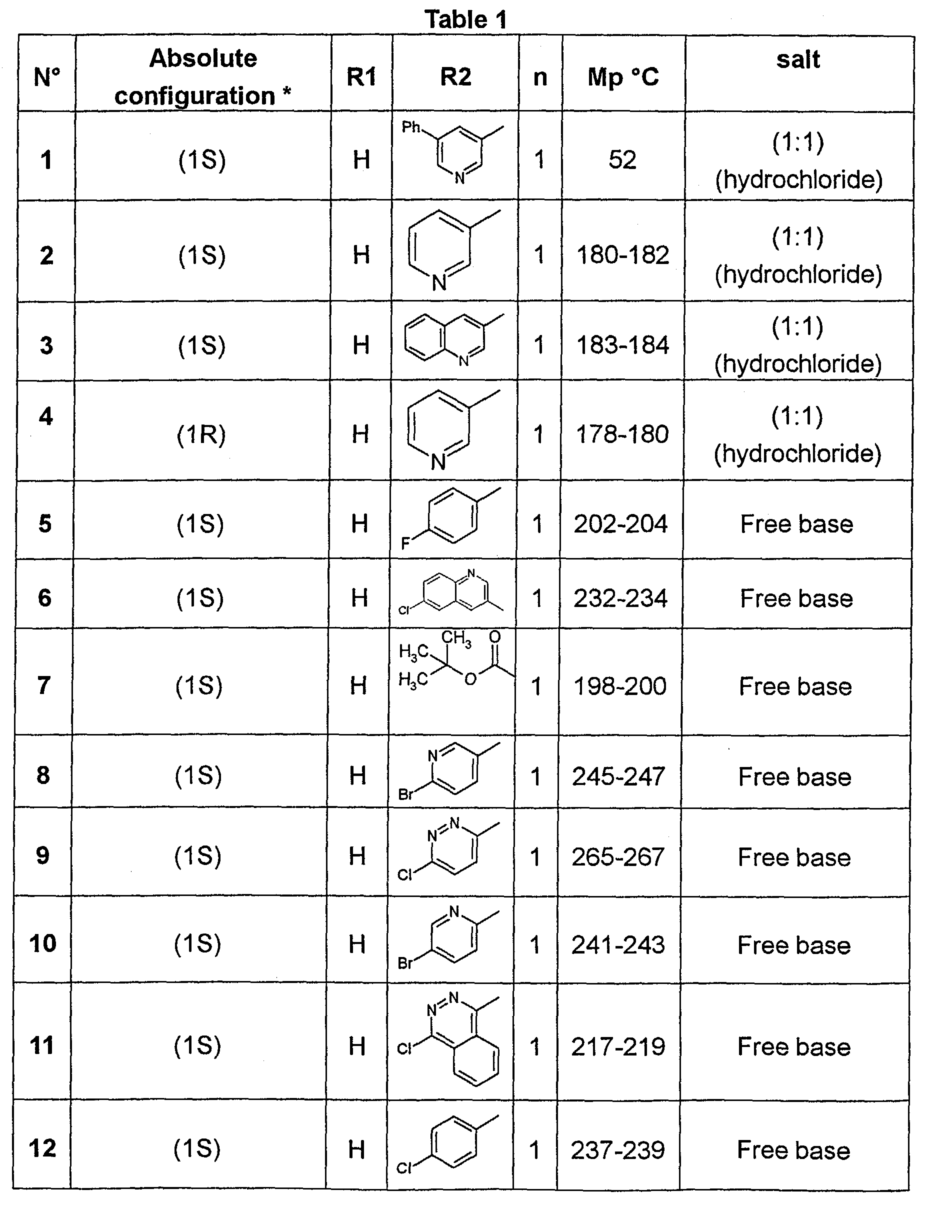

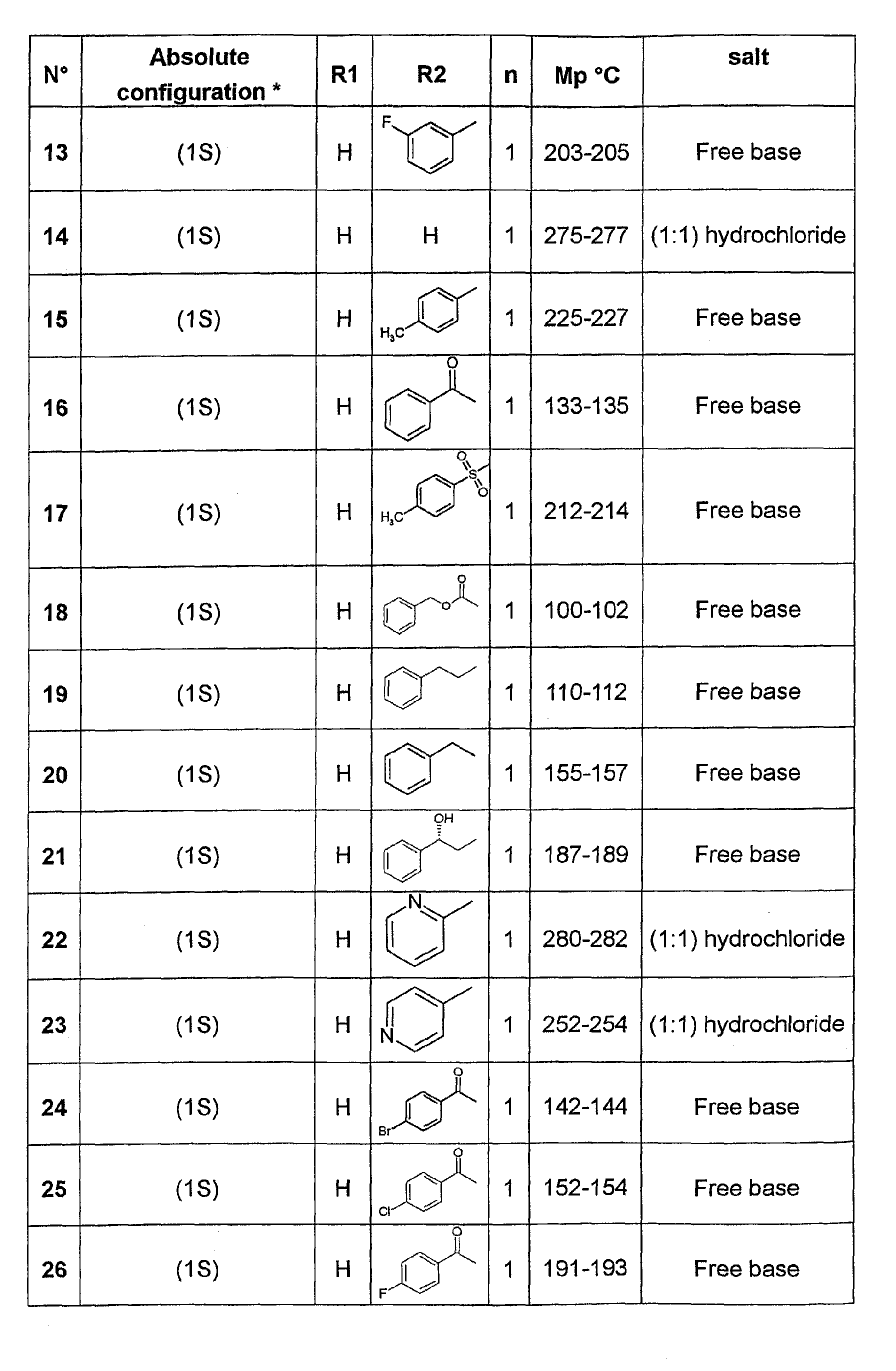

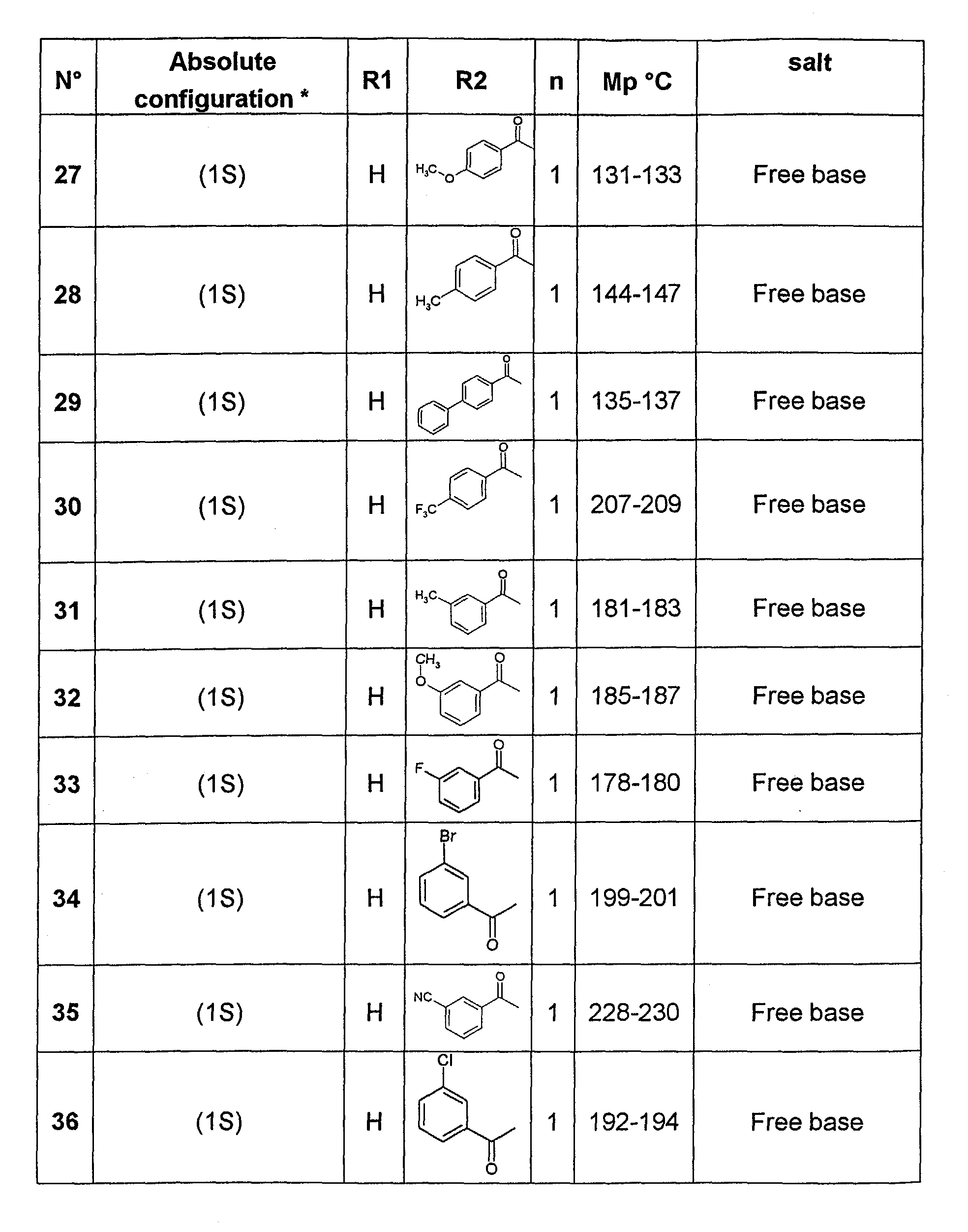

### Test Example: Inhibitory activity of the medicament of the present invention against GSK3β:

Two different protocols can be used.

In a first protocol : 7.5 µM of prephosphorylated GS1 peptide and 10 µM ATP (containing 300,000 cpm of 33P-ATP) were incubated in 25 mM Tris-HCl, pH 7.5, 0.6 mM DTT, 6 mM MgCl₂, 0.6 mM EGTA, 0.05 mg/ml BSA buffer for 1 hour at room temperature in the presence of GSK3beta (total reaction volume : 100 microliters).

In a second protocol: 4.1 µM of prephosphorylated GS1 peptide and 42 µM ATP (containing 260,000 cpm 33P-ATP) were incubated in 80 mM Mes-NaOH, pH 6.5, 1 mM Mg acetate, 0.5 mM EGTA, 5 mM 2-mercaptoethanol, 0.02% Tween 20, 10% glycerol buffer for 2 hours at room temperature in the presence of GSK3beta. Inhibitors were solubilised in DMSO (final solvent concentration in the reaction medium, 1 %).

The reaction was stopped with 100 microliters of a solution made of 25 g polyphosphoric acid (85% P₂O₅), 126 ml 85% H₃PO₄, H₂O to 500 ml and then diluted to 1:100 before use. An aliquot of the reaction mixture was then transferred
to Whatman P81 cation exchange filters and rinsed with the solution described above. Incorporated 33P radioactivity was determined by liquid scintillation spectrometry.
The phosphorylated GS-1 peptide had the following sequence :
NH2-YRRAAVPPSPSLSRHSSPHQS(P)EDEE-COOH.

The GSK3β inhibitory activity of the compounds of the present invention are expressed in IC₅₀, and as an illustration the range of IC₅₀'s of the compounds in table 1 is between 1 nanomolar to 1 micromolar concentrations.

### Formulation Example

### (1) Tablets

The ingredients below were mixed by an ordinary method and compressed by using a conventional apparatus.

| | |
|---|---|
| Compound of Example 1 | 30 mg |
| Crystalline cellulose | 60 mg |
| Corn starch | 100 mg |
| Lactose | 200 mg |
| Magnesium stearate | 4 mg |

### (2) Soft capsules

The ingredients below were mixed by an ordinary method and filled in soft capsules.

| | |
|---|---|
| Compound of Example 1 | 30 mg |
| Olive oil | 300 mg |
| Lecithin | 20 mg |

### (1) Parenteral preparations

The ingredients below were mixed by an ordinary method to prepare injections contained in a 1 ml ampoule.

| | |
|---|---|
| Compound of Example 1 | 3 mg |
| Sodium chloride | 4 mg |
| Distilled water for injection | 1 ml |

### Industrial Applicability

The compounds of the present invention have GSK3β inhibitory activity and are useful as an active ingredient of a medicament for preventive and/or therapeutic treatment of diseases caused by abnormal activity of GSK3β and more particularly of neurodegenerative diseases.

## Claims

1. A 2-(diaza-bicyclo-alkyl)-pyrimidone derivative represented by formula (I) or a salt thereof, or a solvate thereof or a hydrate thereof: 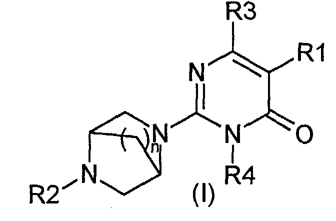 wherein:
R1 represents a hydrogen atom, a C₁₋₆ alkyl group or a halogen atom;
R2 represents a hydrogen atom, a C₁₋₆ alkyl group optionally substituted by 1 to 4 substituents selected from a halogen atom, a hydroxyl group or a C₁₋₄ alkoxy group; a C₁₋₂ perhalogenated alkyl group, a benzyl group, a phenethyl group, a benzyloxycarbonyl group, a C₁₋₄ alkoxy carbonyl group, a benzene ring, a naphthalene ring, a quinoline ring, a phthalazine ring, a 5,6,7,8-tetrahydronaphthalene ring, a pyridine ring, an indole ring, a pyrrole ring, a thiophene ring, a benzenesulfonyl group, a benzoyl group, a pyridazine ring, a furan ring or an imidazole ring; the benzyl group, the phenethyl group, the benzyloxycarbonyl group, the benzenesulfonyl group, the benzoyl group and the rings being optionally substituted by 1 to 4 substituents selected from a C₁₋₆ alkyl group, a benzene ring, a halogen atom, a C₁₋₂ perhalogenated alkyl group, a C₁₋₃ halogenated alkyl group, a hydroxyl group, a C₁₋₄alkoxy group, a nitro, a cyano, an amino, a C₁₋₆ monoalkylamino group or a C₂₋₁₀ dialkylamino group;
R3 represents a 2, 4 or 5-pyrimidine ring optionally substituted by a C₁₋₄ alkyl group, C₁₋₄ alkoxy group or a halogen atom;
R4 represents a C₁₋₄ alkyl group optionally substituted by a hydroxyl group, a C₁₋₄ alkoxy group or a halogen atom and n represents 1 or 2.

2. A 2-(diaza-bicyclo-alkyl)-pyrimidone derivative or a salt thereof, or a solvate thereof or a hydrate thereof according to claim 1, wherein R3 represents an unsubstituted 4-pyrimidinyl group.

3. A 2-(diaza-bicyclo-alkyl)-pyrimidone derivative or a salt thereof, or a solvate thereof or a hydrate thereof according to claim 1 or 2, wherein R2 represents a a hydrogen atom; a benzyl group, a phenethyl group, a benzyloxycarbonyl group, a C₁₋₄ alkoxy carbonyl group, a benzene ring, a quinoline ring, a phthalazine ring, a pyridine ring, a benzenesulfonyl group, a benzoyl group or a pyridazine ring; the benzyl group, the phenethyl group, the benzyloxycarbonyl group, the benzenesulfonyl group, the benzoyl group and the rings being optionally substituted by 1 to 4 substituents.

4. A 2-(diaza-bicyclo-alkyl)-pyrimidone derivative which is selected from the group consisting of:
• (1S)-1-Methyl-2-[5-(5-phenyl-pyridin-3-yl)-2,5-diaza-bicyclo[2.2.1]hept-2-yl]-1*H*-[4,4']bipyrimidinyl-6-one
• (1S)-1-Methyl-2-(5-pyridin-3-yl-2,5-diaza-bicyclo[2.2.1]hept-2-yl)-1*H*-[4,4']bipyrimidinyl-6-one
• (1S)-1-Methyl-2-(5-quinolin-3-yl-2,5-diaza-bicyclo[2.2.1]hept-2-yl)-1*H*-[4,4']bipyrimidinyl-6-one
• (1R)-1-Methyl-2-(5-pyridin-3-yl-2,5-diaza-bicyclo[2.2.1]hept-2-yl)-1*H*-[4,4']bipyrimidinyl-6-one
• (1S)-2-[5-(4-Fluoro-phenyl)-2,5-diaza-bicyclo[2.2.1]hept-2-yl]-1-methyl-1*H*-[4,4']bipyrimidinyl-6-one
• (1 R)-2-[5-(6-Chloro-quinolin-3-yl)-2,5-diaza-bicyclo[2.2.1]hept-2-yl]-1-methyl-1*H*-[4,4']bipyrimidinyl-6-one
• (1S)-5-(1-Methyl-6-oxo-1,6-dihydro-[4,4']bipyrimidinyl-2-yl)-2,5-diazabicyclo[2.2.1 ]heptane-2-carboxylic acid *tert*-butyl ester
• (1S)-2-[5-(6-Bromo-pyridin-3-yl)-2,5-diaza-bicyclo[2.2.1]hept-2-yl]-1-methyl-1*H*-[4,4']bipyrimidinyl-6-one
• (1S)-2-[5-(6-Chloro-pyridazin-3-yl)-2,5-diaza-bicyclo[2.2.1]hept-2-yl]-1-methyl-1*H*-[4,4']bipyrimidinyl-6-one
• (1S)-2-[5-(5-Bromo-pyridin-2-yl)-2,5-diaza-bicyclo[2.2.1]hept-2-yl]-1-methyl-1*H*-[4,4']bipyrimidinyl-6-one
• (1S)-2-[5-(4-Chloro-phthalazin-1-yl)-2,5-diaza-bicyclo[2.2.1]hept-2-yl]-1-methyl-1*H*-[4,4']bipyrimidinyl-6-one
• (1S)-2-[5-(4-Chloro-phenyl)-2,5-diaza-bicyclo[2.2.1]hept-2-yl]-1-methyl-1*H*-[4,4']bipyrimidinyl-6-one
• (1S)-2-[5-(3-Fluoro-phenyl)-2,5-diaza-bicyclo[2.2.1]hept-2-yl]-1-methyl-1*H*-[4,4']bipyrimidinyl-6-one
• (1S)-2-(2,5-Diaza-bicyclo[2.2.1]hept-2-yl)-1-methyl-1*H*-[4,4']bipyrimidinyl-6-one
• (1S)-1-Methyl-2-(5-*p*-tolyl-2,5-diaza-bicyclo[2.2.1]hept-2-yl)-1*H*-[4,4']bipyrimidinyl-6-one
• (1S)-2-(5-Benzoyl-2,5-diaza-bicyclo[2.2.1]hept-2-yl)-1-methyl-1*H*-[4,4']bipyrimidinyl-6-one
• (1S)-1-Methyl-2-[5-(toluene-4-sulfonyl)-2,5-diaza-bicyclo[2.2.1]hept-2-yl]-1*H*-[4,4']bipyrimidinyl-6-one
• (1S)-5-(1-Methy)-6-oxo-1,6-dihydro-[4,4']b)pyrimidinyl-2-yl)-2,5-diazabicyclo[2.2.1]heptane-2-carboxylic acid benzyl ester
• (1S)-1-Methyl-2-(5-phenethyl-2,5-diaza-bicyclo[2.2.1]hept-2-yl)-1*H*-[4,4']bipyrimidinyl-6-one
• (1S)-2-(5-Benzyl-2,5-diaza-bicyclo[2.2.1]hept-2-yl)-1-methyl-1*H*-[4,4']bipyrimidinyl-6-one
• (1S)-2-[5-(2(S)-Hydroxy-2-phenyl-ethyl)-2,5-diaza-bicyclo[2.2.1]hept-2-yl]-1-methyl-1*H*-[4,4']bipyrimidinyl-6-one
• (1S)-1-Methyl-2-(5-pyridin-2-yl-2,5-diaza-bicyclo[2.2.1]hept-2-yl)-1 *H*-[4,4']bipyrimidinyl-6-one
• (1S)-1-Methyl-2-(5-pyridin-4-yl-2,5-diaza-bicyclo[2.2.1]hept-2-yl)-1*H*-[4,4']bipyrimidinyl-6-one
• (1S)-2-(5-(4-Bromo-benzoyl)-2,5-diaza-bicyclo[2.2.1]hept-2-yl)-1-methy)-1*H*-[4,4']bipyrimidinyl-6-one
• (1S)-2-(5-(4-Chloro-benzoyl)-2,5-diaza-bicyclo[2.2.1]hept-2-yl)-1-methyl-1*H*-[4,4']bipyrimidinyl-6-one
• (1S)-2-(5-(4-Fluoro-benzoyl)-2,5-diaza-bicyclo[2.2.1]hept-2-yl)-1-methyl-1*H*-[4,4']bipyrimidinyl-6-one
• (1S)-2-(5-(4-Methoxy-benzoyl)-2,5-diaza-bicyclo[2.2.1 ]hept-2-yl)-1-methyl-1*H*-[4,4']bipyrimidinyl-6-one
• (1S)-2-(5-(4-Methyl-benzoyl)-2,5-diaza-bicyclo[2.2.1]hept-2-yl)-1-methyl-1*H*-[4,4']bipyrimidinyl-6-one
• (1S)-2-(5-(4-Phenyl-benzoyl)-2,5-diaza-bicyclo[2.2.1]hept-2-yl)-1-methyl-1*H*-[4,4']bipyrimidinyl-6-one
• (1S)-2-(5-(4-Trifluoromethyl-benzoyl)-2,5-diaza-bicyclo[2.2.1]hept-2-yl)-1-methyl-1 *H*-[4,4']bipyrimidinyl-6-one
• (1S)-2-(5-(3-Methyl-benzoyl)-2,5-diaza-bicyclo[2.2.1]hept-2-yl)-1-methyl-1*H*-[4,4']bipyrimidinyl-6-one
• (1S)-2-(5-(3-Methoxy-benzoyl)-2,5-diaza-bicyclo[2.2.1]hept-2-yl)-1-methyl-1*H*-[4,4']bipyrimidinyl-6-one
• (1S)-2-(5-(3-Fluoro-benzoyl)-2,5-diaza-bicyclo[2.2.1]hept-2-yl)-1-methyl-1*H*-[4,4']bipyrimidinyl-6-one
• (1S)-2-(5-(3-Bromo-benzoyl)-2,5-diaza-bicyclo[2.2.1]hept-2-yl)-1-methyl-1*H*-[4,4']bipyrimidinyl-6-one
• (1S)-2-(5-(3-Cyano-benzoyl)-2,5-diaza-bicyclo[2.2.1]hept-2-yl)-1-methyl-1*H*-[4,4']bipyrimidinyl-6-one and
• (1S)-2-(5-(3-Chloro-benzoyl)-2,5-diaza-bicyclo[2.2.1]hept-2-yl)-1-methyl-1*H*-[4,4']bipyrimidinyl-6-one;
or a salt thereof, or a solvate thereof or a hydrate thereof.

5. A medicament comprising as an active ingredient a substance selected from the group consisting of a 2-(diaza-bicyclo-alkyl)-pyrimidone derivative represented by formula (I) or salts thereof, or a solvate thereof or a hydrate thereof according to claim 1.

6. A GSK3β inhibitor selected from the group of a 2-(diaza-bicyclo-alkyl)-pyrimidone derivative represented by formula (I) or salts thereof, or a solvate thereof or a hydrate thereof according to claim 1.

7. Use of a compound according to any one of claims 1 to 4 for the preparation of a medicament for preventive and/or therapeutic treatment of a disease caused by abnormal GSK3β activity.

8. Use of a compound according to any one of claims 1 to 4 for the preparation of a medicament for preventive and/or therapeutic treatment of a neurodegenerative disease.

9. Use of a compound according to claim 8, wherein the neurodegenerative disease is selected from the group consisting of Alzheimer's disease, Parkinson's disease, tauopathies, vascular dementia; acute stroke, traumatic injuries; cerebrovascular accidents, brain cord trauma, spinal cord trauma; peripheral neuropathies; retinopathies or glaucoma.

10. Use of a compound according to claims 1 to 4 for the preparation of a medicament for preventive and/or therapeutic treatment of non-insulin dependent diabetes; obesity; manic depressive illness; schizophrenia; alopecia; or cancer.

11. Use according to claim 10 wherein cancer is breast cancer, non-small cell lung carcinoma, thyroid cancer, T or B-cell leukemia or virus-induced tumors.
